# EUROPEAN PATENT SPECIFICATION

(11) **EP 0 375 647 B1**
(45) Date of publication and mention of the grant of the patent: **09.11.1994**
(21) Application number: 89850444.4
(22) Date of filing: 19.12.1989
(51) Int. Cl.: A61K 6/06, A61K 6/027, A61C 8/00

(54) **Artificial tooth crown**
Künstliche Zahnkrone
Couronne dentaire artificielle

(30) Priority: 20.12.1988 SE 8804588
(43) Date of publication of application: 27.06.1990
(73) Proprietor: SANDVIK AKTIEBOLAG, 811 81 Sandviken (SE)
(72) Inventor: Odén, Agneta, S-182 74 Stocksund (SE); Rostvall, Thomas, S-126 52 Hägersten (SE)
(74) Representative: Östlund, Alf Olof Anders

(56) References cited:
- EP-A- 0 030 850
- EP-A- 0 254 470
- EP-A- 0 337 309
- DE-A- 2 447 787
- US-A- 4 321 042

## Description

This invention relates to accurate shaping of artificial all ceramic tooth crowns for dental prosthetic constructions. By starting from a prefabricated coping a tooth crown is obtained which is easier to produce and to low cost.and the crown has higher strength and accuracy to shape. Preferably the coping is manufactured by a biocompatible, high strength, ceramic material, which is sintered to high density.

U.S Patent 4,575,340 describes a prefabricated abutment used in combination with a prefabricated sleeve-like coping telescopically mated to the abutment. This coping is made with a known lost-wax method in a precious or non-precious metal.

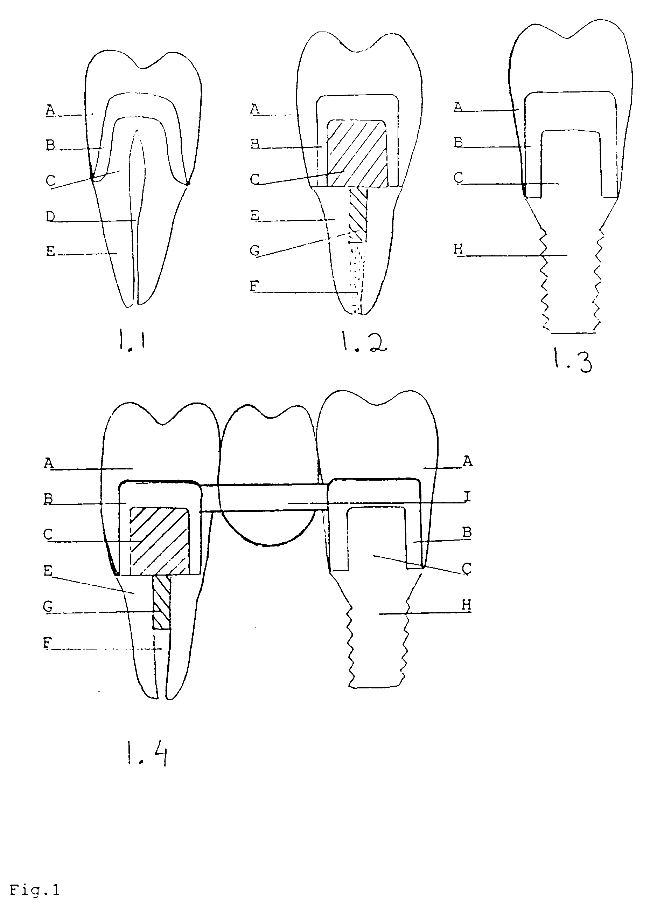

Fig.1 shows different alternative designs of artificial tooth crowns on natural root (1.1), on filled root (1.2), on implant (1.3) and on bridge construction (1.4). In this figure A=veneer, B=coping, C=abutment, D= pulp, E=natural tooth root, F= root filling, G= endodontic post, H= root implant, I = beam with a pontic. It appears from the figure that artificial tooth crowns are designed as a coping (B) with veneer (A). They are fixed on an abutment (C) with cement or a mechanical joint. The crowns are made from different materials such as metal, porcelain, glass or combinations thereof, and plastic can also be used.

Artificial tooth crowns are today manufactured mainly in the following way: A dentist makes a preparation on a tooth, on which a tooth crown is to be fixed in the mouth of a patient, an impression is made and with this impression a copy of the preparation is made in gypsum. On this model a dental technician prepares a crown in wax. The adjacent teeth must be considered, and the dental technician must have models from the two jaws. A sprue former in wax is fixed on one of the cusps of the wax crown. The wax crown is loosened from the gypsum model. The wax crown with the sprue former are invested in a metal ring with investment. The wax is burnt out and a crown can be cast in a precious or non-precious metal. The cast crown can in certain cases be covered by a veneer made of porcelain in order to obtain a colour of the tooth crown similar to the colour of natural teeth. Instead of porcelain plastic material can be used.

The fabrication of tooth crowns in glass is very close to the technique described above with the difference that after the casting a thin layer of porcelain is painted on the surface and fired, in order to give the tooth crown individual tooth colours.

Tooth crowns fabricated mainly of porcelain can be made with conventional dental porcelain technique on a sheet made of a precious or non-precious alloy. Porcelain crowns can also be made with conventional dental porcelain technique on a model of the abutment. The material in this model has no changes in dimension on heating up to 1200^{o}C. When the tooth crown is ready the model of the abutment is removed by sand blasting.

The above described complicated and time consuming methods are today used to manufacture crowns, which will fit on individually prepared natural teeth, on prefabricated abutments for implants, and on prefabricated pins for anchoring of tooth crowns to root filled teeth.

The problem with the material now used (porcelain, glass etc) in artificial tooth crowns is their brittleness, which often gives early fracture, and these artificial crowns must be replaced more or less regularly.

The object of the present invention is to provide an artificial all ceramic tooth crown, which is easier and accordingly cheaper to make and, in addition, has higher strength and accuracy to shape.

Another purpose is to make a crown by using densely sintered, high strength ceramic material, provided that the demand of high strength, accuracy to shape (that means compensate for the shrinking during sintering) can be combined with the demand on the application of porcelain concerning burning, adherence, and esthetics.

According to the invention artificial tooth crowns now exist, in which the coping is prefabricated in a biocompatible material i.e. the internal cavity has been given such dimensions as to fit on an abutment, which can be a part of an implant, a root canal pin or a part of a bridge. A biocompatible material for copings is a material which is not toxic and does not cause damage on oral tissues or does not give unwanted system effects. In addition this material must not give any discolourations or other unwanted effects to the veneer material. The crown is fixed by cementation or by a mechanical joint e.g. screw joint.

The invention allows a considerable simplification of the handicraft of the dental technician. With the aid of gypsum models of the two jaws and with the prefabricated coping on the model of the abutment the dental technician can make the final design of the tooth crown and at the same time control its function and size. In order to make the veneer a porcelain furnace or an apparatus for pressing of composite veneer is needed. The method to make a tooth crown according to the invention to an existing abutment, decreases essentially the time of production for tooth crowns and at the same time the strength and precision regarding the accuracy to shape increase.

Copings according to the invention can be made of metals or alloys by e.g. casting according to the method described above. They can also be made by fine mechanical machining such as turning, milling, drilling, spark erosion etc. Alternatively the copings can be made by pressing a material in sheet . They can also be made in porcelain or glass where applicable according to the methods described above.

Preferably the coping according to the invention is made from a densely sintered, high strength ceramic material with powder metallurgical methods. It has been found that it is possible to compact and sinter a ceramic powder ready to press to a coping for artificial tooth crowns. Suitably the coping is ground before the sintering so that only smaller adjustments are needed after the sintering. On the obtained coping the veneer is built up, so that the final product will be a tooth crown fitting to existing abutment and to the actual whole set of teeth.

The ceramic powder can be made by several for the expert well known methods. Traditional powder metallurgical technique can be used, where the different components are mixed and ground under dry or wet conditions with water or an inorganic solvent (e.g.alcohols) as grinding liquid. So called SOL-GEL technique can also be used where different oxide materials are deposited together from a water solution or are coprecipitated from metal alcoxides in e.g. water free alcohol by controlled addition of water. Combination of different techniques can also be used by using SOL-GEL technique to deposit a surface layer of desired metal oxide on a powder material. To the ceramic powder pulp lubricants or other organic binders depending on the choice of forming method are added, when needed at suitable time in the process.

Other preparation routes of the ceramic material are also possible as reaction sintering where a suitable metal is oxidized, nitrided etc. For example, aluminium/alumina can be oxidized under carefully controlled processing to alumina. These methods allow preforming or reinforcement by fibres, e.g. in a filt infiltrated with liquid metal.

Many of the monolithic ceramics which are biocompatible might have a brittle performance if they are not sintered to nearly full density, more than 98% and preferably > 99.5% of the theoretical density. However, these ceramics can be strenghtened by a number of toughening mechanisms. Finely dispersed particles, platelets, whiskers or fibers raise the fracture toughness of the composite. Typical additives are the nitrides, carbides, borides or mixtures thereof of the transition metal oxides of group IV-VI or of the elements Al or Si. Toughening may also be achieved by so called transformation toughening, i.e. additions of unstabilized ZrO₂ or ZrO₂ stabilized with Y₂O₃, MgO or CaO. The additions of these latter oxides shall not exceed 25 wt%, but should be more than 2 wt%. The best performance is obtained with 3-12 wt%.

According to the invention artificial ceramic tooth crowns now exist characterized in that they are made from a ready to press powder with additions of lubricants and/or other organic binders. The powder is cold isostatically compacted, uniaxially pressed, slip cast, pressure cast, injection moulded or compacted in another way. The compacted body has such dimensions that it after the shrinking during the subsequent sintering process to high density, with great accuracy has desired final geometrical internal shape. It is important that the ceramic material is sintered to closed porosity, which for an oxide material means at least 95% of theoretical density, but in order to ensure good mechanical strength the material should preferably have a density over 98%, while density over 99.5% gives the best strength.

The sintering takes place in vacuum, under normal atmospheric pressure or under increased pressure in connection with overpressure sintering or hot isostatic compaction, alternatively hot pressing. Pure oxide materials like Al₂O₃, TiO₂_{,} MgO, ZrO₂, MgAl₂O₄_{,} Al₂TiO₅ etc. or SiO₂ and silicates, can be sintered in air, but some composites have to be sintered in inert or controlled atmosphere. The coping is given an external shape so that the following building-up of the veneer is facilitated. The external shape can e.g. be cylindrical or slightly cone-shaped. The cross section can be e.g.circular, oval or angular. The shape can also be such that it is roughly similar to natural teeth. If dental porcelain is used as veneer with a coefficient of thermal expansion adapted to the material in the coping this porcelain will adhere better to the coping. In the case of Al₂O₃ or other glass forming oxides there will be a "chemical bond " between Al₂O₃ and porcelain. This means that the external surface of the coping does not need any retention elements. When using other veneer materials e.g. plastic, mechanical retention elements can be needed e.g. grooves, pits or on the coping sintered retention elements.

Preferably the ceramic copings are made in large scale production by uniaxial compaction. Therewith a mandrel is used uniform to the artificial abutment on which the crown will fit. The mandrel, which will be made from a hardenable steel and is hardened after manufacturing, has such a size that the coping after the sintering shrinkage will have the desired fit to the natural or artificial abutment in question. During the compaction the mandrel is placed on the ejector and will give the internal shape to the coping, that means the shape which will fit to the prefabricated abutment. The external shape of the coping can be made during the compaction by the upper and lower punches, but the external shape can also be made by grinding preferably before the sintering.

The ceramic base material in the coping comprises preferably one or several biocompatible oxides (including phosphates, silicates and sulfates), with additives of carbides, silicides, nitrides or borides with or without binder metal in addition to conventional sintering aids. The oxide mixtures can comprise single oxide phases like Al₂O₃ + MgO or partly or fully reacted new oxides like MgAl₂O₃. Similarly the presence of SiO₂ addition can after sintering form both a glassy phase or crystalline phases like remanent SiO₂ or formed silicates. The base material can also comprise other high performance ceramics which are biocompatible; such as nitrides, oxynitrides, carbides etc. Examples of the two former materials are Si₃N₄, Si₂N₂O, sialon, AlN, AlON etc. Examples of biocompatible oxides, which can form base matrix for the ceramic body, are refractory oxides like Al₂O₃, TiO₂, MgO, SiO₂, ZrO₂ and ZrO₂ with additives of smaller amounts of up to 10 mol% of Y₂O₃ or MgO (partly or totally stabilized ZrO₂). Included componenets can be present as particles with a size of < 25µm preferably <10µm and/or as whiskers (hair shaped single crystals) with a length to diameter ratio>5, preferably >10 and/or fibers (polycrystalline) with a diameter of >10µm and/or as single crystal platelets with an approximate diameter of 5-50 µm, preferably 5-20 µm and a thickness of 1-10µm, preferably 1-4 µm. The amount of particles, whiskers, fibers and/or platelets should not exceed 60 volume%, preferably less than 40 vol %. Examples of these included components are SiC, TiN, TiC, TiB₂, Si₃N₄, or other biocompatible carbides or nitrides of the transition metal group IV, V or VI.

In a preferred embodiment the ceramic material comprises >50% preferably >85% of Al₂O₃ with additives of conventional sintering aids. In order to increase the strength < 25 weight % preferably 3-12 weight %of ZrO₂, and/or 5-40 weight % preferably 10-30 weight % of SiC whiskers can be added. In order to get a suitable colour, coloured components can be chosen. Additives e.g 0.1-10 weight % preferably 0.5-5 weight % of TiN and/or ZrN will give Al₂O₃ based copings a faint, yellow shade.

For some purposes it can be suitable to coat the coping before the veneering with at least one thin layer 1-10µm of e.g.Al₂O₃ or TiN. The coating is performed with in itself known technique e.g. Chemical Vapour Deposition (CVD) or Physical Vapour Deposition (PVD).

### Example

Copings to an abutment with a hexagonal shape of titanium for implants, height=4 mm and distance between parallel sides=3.3 mm, were uniaxially compacted against a mandrel with a shape uniform to the abutment. The mandrel had such a size that it allowed a shrinkage of 17% during the sintering. It was placed as ejector to a cylindrical tool with a diameter of 6.2 mm. The powder used for the compaction had the approximate composition 95.75 weight% of Al₂O₃ , 4 weight% of ZrO₂ and 0.25 weight% of MgO and a powder with 99.75% of Al₂O₃ and 0.25% of MgO resp. The sintering was performed in air during 2 hours at 1600^{o}C. After the sintering the blanks had a relative density of 99.5% and the following external dimension: diameter=5.1 mm and height=8.9 mm. The blanks were ground cylindrical with mandrel down to a diameter of 4.8 mm and commercially available dental porcelain was fired on the surface. The first layer porcelain contained about 50% Al₂O₃ and was fired at 1150^{o}C during 15 minutes. During the heating the furnace was under vacuum, but when the final firing temperature was reached the firing was performed under atmospheric pressure. The remainder of the crown was fired at 1050^{o}C. The dental porcelain combined chemically with the alumina without any gap between the porcelain and the densely sintered coping. The crowns fitted perfectly on the abutment and were ready to be cemented with conventional methods on abutment in the mouth of a patient.

## Claims

1. Artificial tooth crown comprising a coping and a veneer, wherein the coping is prefabricated of a biocompatible ceramic material comprising > 50 % by weight of Al₂O₃ **characterized** in that the coping is formed of a sintered material which shrinks during sintering and shows a closed porosity after sintering.

2. Artificial tooth crown according to claim 1 **characterized** in that the ceramic material also includes one or more of the group consisting of TiO2, MgO, ZrO2 or ZrO2 stabilized with up to 10 mol-% Y2O3 or MgO.

3. Artificial tooth crown according to any of the preceding claims **characterized** in that the coping also comprises whiskers and/or particles of SiC, TiN, ZrO2 and/or ZrN.

## Patentansprüche

1. Künstliche Zahnkrone mit einer Kappe und einem Überzug, wobei die Kappe aus einem biologisch verträglichen Keramikmaterial vorgefertigt ist, welches >50 Gew.-% Al₂O₃ umfaßt, **dadurch gekennzeichnet**, daß die Kappe aus einem gesinterten Material besteht, welches während des Sinterns schrumpft und eine geschlossene Porosität nach dem Sintern zeigt.

2. Künstliche Zahnkrone nach Anspruch 1, **dadurch gekennzeichnet**, daß das Keramikmaterial auch einen oder mehrere Stoffe der Gruppe einschließt, die aus TiO₂, MgO, ZrO₂ oder mit bis zu 10 mol-% Y₂O₃ oder MgO stabilisiertem ZrO₂ besteht.

3. Künstliche Zahnkrone nach einem der vorausgehenden Ansprüche, **dadurch gekennzeichnet**, daß die Kappe auch Whisker und/oder Teilchen von SiC, TiN, ZrO₂ und/oder ZrN umfaßt.

## Revendications

1. Couronne dentaire artificielle comprenant une copie et un placage, dans laquelle la copie est préfabriquée en une matière céramique biocompatible comprenant plus de 50% en poids de Al₂O₃, caractérisé en ce que la copie est formée d'une matière frittée qui se rétracte pendant le frittage et présente une porosité fermée après frittage.

2. Couronne dentaire artificielle selon la revendication 1, caractérisée en ce que la matière céramique comprend également un ou plusieurs éléments du groupe constitué de TiO₂, MgO, ZrO₂ et ZrO₂ stabilisé avec jusqu'à 10% en moles de Y2O₃ ou MgO.

3. Couronne dentaire artificielle conformément à l'une quelconque des revendications précédentes, caractérisée en ce que la copie comprend également des moustaches et/ou des particules de SiC, TiN, ZrO₂ et/ou ZrN.
